# EUROPÄISCHE PATENTSCHRIFT

(11) **EP 3 248 564 B1**
(45) Veröffentlichungstag und Bekanntmachung des Hinweises auf die Patenterteilung: **27.10.2021**
(21) Anmeldenummer: 17173168.0
(22) Anmeldetag: 08.02.2010
(51) Int. Cl.: A61C 1/00, A61B 18/22, A61C 1/18

(54) **LASERHANDSTÜCK MIT EINEM AUSWECHSELBAREN GLASFASEREINSATZ**
LASER HANDPIECE WITH AN EXCHANGEABLE GLASS FIBRE INSERT
PIÈCE À MAIN LASER AVEC UN INSERT EN FIBRE DE VERRE INTERCHANGEABLE

(30) Priorität: 06.02.2009 DE 102009000685
(43) Veröffentlichungstag der Anmeldung: 29.11.2017
(62) Teilanmeldung aus: 10152860.2
(73) Patentinhaber: Sirona Dental Systems GmbH, 64625 Bensheim (DE)
(72) Erfinder: BIERBAUM, Thomas, 64625 Bensheim (DE); GOISSER, Siegfried, 64683 Einhausen (DE); LANDGRAF, Hermann, 64653 Lorsch (DE); REIN, Matthias, 64653 Lorsch (DE); SUTTER, Ralf, 69469 Weinheim (DE); PEUCKERT, Joachim, 74357 Bönnigheim (DE); JERGER, Thomas, 72116 Mössingen (DE)
(74) Vertreter: Özer, Alpdeniz

(56) Entgegenhaltungen:
- EP-A1- 0 882 432
- DE-T2- 69 327 187
- JP-A- 2005 015 111
- US-A- 5 976 124
- US-A- 6 102 926
- US-A1- 2005 154 379
- US-A1- 2007 191 823

## Beschreibung

### Technisches Gebiet

Die Erfindung betrifft ein Laserhandstück, einen auswechselbaren Glasfasereinsatz und eine Steuereinheit hierfür. Für die Bearbeitung von Zähnen oder Zahnfleisch in der Mundhöhle eines Patienten ist es bekannt, Laserlicht in einer Lasereinheit zu erzeugen und in den Patientenmund zu führen und auf die zu bearbeitende Stelle auszurichten.

### Stand der Technik

Dafür sind im wesentlichen zwei verschiedene Ausführungen bekannt. So ist eine bis zu 3 m lange Glasfaser am proximalen Ende mit einer Lasereinheit verbunden und das distale Ende wird zur Behandlung eingesetzt. Die Glasfaser wird nach jeder Anwendung aus hygienischen Gründen um ein definiertes Maß gekürzt. Sobald eine gewisse Mindestlänge des Abstands zwischen der Lasereinheit und dem Patient unterschritten wird, ist die restliche Glasfaser vollständig zu erneuen. Zur Führung des distale Endes der Glasfaser dient das Handstück, das auf die Glasfaser aufgeschoben wird und nach jedem Kürzen der Glasfaser an dieser entlang zurückgeschoben wird.

Weiterhin ist es bekannt, die Lasereinheit über einen Lichtleiter mit einem Handstück zu verbinden. Innerhalb des Handstückes erfolgt eine Lichtübertragung von dem Lichtleiter zu einer Glasfaser, welche zur Behandlung am Patienten eingesetzt wird. Diese Glasfasern werden heute zur Einmalverwendung angeboten und sind daher auswechselbar im Handstück befestigt.

In beiden Fällen erfolgt die Aktivierung der Lasereinheit und somit die Aktivierung des Laserstrahles über einen Fußschalter, wie in der Zahnarztpraxis üblich.

Aus der DE 693 27 187 T2 ist ein Laserhandstück bekannt, dem Laserstrahlung von einer externen Laserquelle zu einer wegwerfbaren oder verbrauchbaren optischen Faser aufweist. Diese Einweg- oder Wegwerffaser ist mit dem Handstück verbunden, wobei auf optische Komponenten und insbesondere Linsen zwischen einer in dem Handstück installierten optischen Faser zur Ausgabe von Laserstrahlung an das Handstück und einer austauschbaren optischen Ausgabefaser verzichtet werden kann, da eine zusätzliche Kühlung der Übergabestelle stattfindet.

Es sind weiterhin Ausführungen bekannt, bei denen ein Fingerschalter zur Aktivierung der Laserstrahlung dem Handstück zugeordnet sind.

Für die vorgesehene Wiederverwendung der Glasfaser lassen sich Verschmutzungen der Glasfaser, insbesondere im Bereich des für die Behandlung verwendeten und im Körperkontakt stehenden distalen Endes der Glasfaser auf einer letzten Länge von einigen Millimetern, hervorgerufen durch Karbonisierung von Körpergewebe nicht ohne Funktionsbeeinträchtigung beseitigen, sodass typischerweise die Glasfaser gekürzt wird. Die Ausführung mit einem auf der Glasfaser angeordneten verschiebbaren Handstück hat insbesondere in Verbindung mit einem elektrischen Fingerschalter den Nachteil, dass die Länge des Glasfaser von Anwendung zu Anwendung abnimmt, während die Länge der elektrischen Leitung typischerweise konstant bleibt. Eine Funkübertragung des Schaltsignals ist kompliziert, platzaufwändig und erfordern einen Energiespeicher. Besondere Bedeutung hat hierbei die Sicherheit, da eine mögliche Fehlaktivierung oder eine Nichtabschaltung des Lasers zu einer sehr erheblichen Gefährdung des Patienten, des Anwenders oder des Hilfspersonals führt.

US 5,976,124 offenbart eine Vorrichtung und Verfahren zur Phototherapie. US 2007/0191823 A1 offenbart eine Beleuchtete Lasersonde mit einstellbarem Beleuchtungsbereich.US 2005/0154379 A1 offenbart eine justierbare Lasersonde zur Verwendung in der vitreoretinalen Chirurgie. US 6,102,926 offenbart eine Vorrichtung zur perkutanen Durchführung einer myokardialen Revaskularisation mit Mitteln zur Erfassung von Gewebeparametern und Verfahren zur Anwendung. EP 0 882 432 A1 offenbart ein Zahnärztliches Handstück. JP 2005 015111 A1 offenbart ein Verfahren zur Lagerung großer körper. DE 693 27 187 T2 offenbart ein Handstück zum Abgeben einer Laserbestrahlung.

Die Aufgabe der Erfindung besteht darin, ein Laserhandstück mit einer mehrfach verwendbaren Applikationselement bereitzustellen, wobei die Länge des Handstücks und der optischen und elektrischen Zuleitung zwischen Handstück und der Steuereinheit und damit auch die Behandlungsergonomie selbst möglichst unverändert ist.

Eine weitere Aufgabe besteht darin, eine auswechselbare Glasfaser bereit zu stellen, die einfach transportiert, sterilisiert und montiert werden kann.

Nach eine weitere Aufgabe besteht darin, ein Steuergerät für ein Laserhandstück so auszubilden, dass der Lichtleiter für die Zuführung von Laserlicht zu dem Handstück bequem aufgewickelt und gehalten werden kann.

### Darstellung der Erfindung

Diese Aufgaben werden mit den unabhängigen Ansprüchen gelöst, vorteilhafte Weiterbildungen sind in den Unteransprüchen angegeben.

Ein erfindungsgemäßes Laserhandstück umfasst einen Lichtleiter, bei dem der Lichtleiter an einer Lichteinkopplungsstelle in einem Grundkörper befestigt ist und bei dem ein Applikationselement für Laserlicht auswechselbar an dem Grundkörper befestigt ist. Der Grundkörper ist dabei in einer Griffhülse axial verschiebbar gehalten. Unter Lichtleiter wird hier eine Zuleitung von einem Laserlicht bereitstellenden Steuergerät verstanden, ein Applikationselement dient hingegen für eine Bearbeitung mit Laserlicht.

Das Grundkonzept einer Griffhülse mit einem darin verschiebbaren Grundkörper erlaubt die Längenanpassung des Abstandes des distalen Endes des Applikationselementes, in der Regel einer Glasfaser, zum in der Regel durch eine Führungskanüle, auch als Führungsröhrchen bezeichnet, gebildeten distalen Ende der Griffhülse. Diese Längenanpassung ist wünschenswert, da unterschiedliche Anwendungen eine unterschiedliche Länge erfordern. Beispielsweise wird für chirurgische Behandlungen in der Regel eine freie Länge der Glasfaser von ca. einem Zentimeter ausreichend sein, bei Wurzelkanalbehandlungen werden Längen von zwei bis drei Zentimetern gefordert. Ein besonderer Vorteil besteht darin, dass die Halteposition des Laserhandstücks bezüglich der Führungskanüle von der Länge der Glasfaser unabhängig ist und der Anwender so immer die gleiche, gewohnte Position einnehmen kann.

Vorteilhafterweise kann die Griffhülse mehrteilig sein und einen Fingerauflagebereich, einen Stützbereich und einen Verstellbereich aufweisen. Durch die Aufteilung der Griffhülse in verschiedene Bauteile kann eine einfache Montage erreicht werden.

Um die Verschiebung zu begünstigen, kann der Grundkörper einen durch die Griffhülse hervorstehenden Verschiebeknopf aufweisen, wobei die Griffhülse eine Verschiebebahn mit einer Führung für den Verschiebeknopf aufweisen kann.

Dabei kann der Verschiebeknopf gegen eine Federkraft von einer ersten, gehemmten Stellung in eine zweite Verschiebestellung bringbar sein. Hierdurch wird ein unbeabsichtigtes Verstellen vermieden.

Vorteilhafterweise kann am Handstück ein Tastschalter zum Anschalten des Laserlichts vorgesehen sein, wofür sich eine Anordnung im Fingerauflagebereich besonders anbietet. Insbesondere dann, wenn ein in Längsrichtung verlaufendes langen Tastenfeld vorgesehen ist, dessen Länge vorzugsweise zwischen 20 mm und 45 mm betragen kann, können auch in Längsrichtung gesehen unterschiedliche Haltepositionen des Laserhandstücks bereitgestellt und eine Betätigung des Tastschalters sichergestellt werden.

Von besonderer Bedeutung für die Erfindung ist die Möglichkeit, den Tastschalter am Grundkörper selbst vorzusehen, da dann an der Griffhülse selbst keine elektrischen Bauteile vorhanden sein müssen und die Griffhülse vielfach und insbesondere mit Heißdampf sterilisiert werden kann. Dabei kann der Tastschalter von einem an der Griffhülse angebrachten schwenkbaren Hebel abgedeckt sein, wobei vorzugsweise die Länge des Hebels zur Schwenkachse mindestens das 2-fache, besonders vorzugsweise das 3- bis 5-fache der über die Griffhülse bereitgestellten Betätigungslänge zur Auslösung des Tastschalters beträgt. Unter Betätigungslänge wird die Länge verstanden, bei der beim Einwirken auf die Griffhülse noch eine Auslösung des Tastschalters sichergestellt ist. Diese Überlänge des Hebels gegenüber der Betätigungslänge stellt sicher, dass zum einen nur geringe Unterschiede beim Schaltweg und bei der Schaltkraft vorhanden sind.

Die besondere Ausbildung mit einem auf den Tastschalter wirkenden Hebel hat den Vorteil, dass der oder im Falle mehrerer die Tastschalter über den ganzen Verschiebeweg mit nahezu der gleichen Kraft betätigt werden können. Damit lässt sich sowohl ein großes Tastenfeld bereitstellen als auch die Betätigung der Tastschalter in jeder Lage des Grundkörpers relativ zur Hülse sicherstellen.

Der Tastschalter am Grundkörper kann gegen unbeabsichtigtes Betätigen bei abgezogener Hülse gesichert sein. Dazu kann der Tastschalter beispielsweise in einer Vertiefung am Grundkörper angeordnet sein oder in der unmittelbaren Nachbarschaft des Tastschalters können Erhöhungen vorgesehen sein.

Ein versehentliches Auslösen durch den Anwender ist nach dem Stand der Technik dann nicht möglich, wenn die Gestaltung derart erfolgt, dass der in dem anzuwendenden Standard IEC 60601-1 beschriebene Normfinger eine Betätigung nicht erlaubt. Die mechanische Sicherung des Tastschalters kann dabei durch eine Erhebung, vorzugsweise durch in Längsrichtung verlaufende seitliche Stege gebildet sein, deren Höhe gegenüber den Tastschaltern und deren Abstand zueinander eine Betätigung des Tastschalters durch einen Normfinger verhindert.

Zum Anschalten des Laserlichts kann der Tastschalter mit einer Auswerteeinheit verbunden werden, etwa über einen elektrischen Schaltkreis oder über einen Signalleiter, der auch auf optischem Weg Signale übertragen kann.

Zur Erhöhung der Betriebssicherheit können mehrere redundante Tastschalter vorgesehen sein.

Eine erfindungsgemäße Weiterbildung besteht darin, dass der Grundkörper einen auf den Lichtweg im Grundkörper ausgerichteten Sensor aufweist, der Licht in der Wellenlänge des über den Lichtleiter zugeführten Lichts erkennt und der wie auch für die Tastschalter vorgesehen ein Signal an eine Auswerteeinheit übermitteln kann, die im Laserhandstück oder in einer Steuereinheit vorhanden ist. Insbesondere beim Betrieb mit einem auswechselbaren Glasfasereinsatz erhöht die Erfassung des Vorhandenseins dieses auswechselbaren Glasfasereinsatzes mit einem Sensor die Sicherheit beim Betreiben des Laserhandstücks. Darüber hinaus kann aber auch ein Bruch der Glasfaser im Inneren der Griffhülse erkannt werden.

Zur Erhöhung der Betriebssicherheit können mehrere redundante Sensoren vorgesehen sein.

Das Laserhandstück kann ein auswechselbares Applikationselement für Laserlicht aufweisen, dessen dem Bearbeitungsende abgewandtes, proximales Ende an einer Lichteinkoppelstelle in dem Grundkörper befestigt ist. Das Applikationselement kann dabei insbesondere ein auswechselbarer Glasfasereinsatz sein.

Dieser auswechselbarer Glasfasereinsatz kann sterilisierbar sein und die Teile mit Patientenkontakt können ebenfalls sterilisierbar ausgeführt werden. Selbstverständlich können zur Vervollständigung der notwendigen Maßnahmen auch automatisierte Verfahren zur Reinigung, Desinfektion und Sterilisation eingesetzt werden. Die Vorsehung von austauschbaren Glasfasereinsätzen verringert die Behandlungskosten, wobei sichergestellt ist, dass die eigentliche Handstücklänge immer gleich ist. Trotz der erforderlichen Kürzung der Glasfaser aufgrund von Abnutzung oder Verschmutzung durch eingebranntes Gewebe ist eine mehrfach Nutzung der Glasfaser möglich, da die gekürzte Länge ausgeglichen werden kann.

Vorteilhafterweise kann der Lichtleiter in einem Handstückschlauch untergebracht sein, wobei der Handstückschlauch mit dem Grundkörper verbunden ist und gegenüber der Griffhülse frei ist. Zusätzlich kann auch eine elektrische Leitung des elektrischen Schaltkreises oder eine andere, etwa optische Signalleitung vorhanden sein, die zusammen mit dem Lichtleiter zu einem Steuergerät geführt werden.

Ein weiterer Gegenstand der Erfindung ist ein auswechselbarer Glasfasereinsatz für ein Laserhandstück, aufweisend ein an einem Ende angeordnetes Anschlussstück zum Anschluss an das Laserhandstück und eine sich von dem Anschlussstück weg erstreckende Glasfaser mit einem dem Anschlussstück abgewandten Bearbeitungsende, wobei das Anschlussstück einen Abschnitt zur Einleitung und Übertragung eines Drehmoments auf eine weiterer Halteabschnitt aufweist. Weiterhin ist eine die Glasfaser umgebende Hülse vorgesehen, die auf den Drehmomentübertragungsbereich des Anschlussstücks aufsteckbar ist und deren Ende einen Verbindungsbereich aufweist, der mit dem Drehmomentübertragungsbereich des Anschlussstücks zusammenwirkt.

Die Aufsteckhülse mit gleichzeitiger Werkzeugausformung dient einerseits als Transportschutz für die Glasfaser, als Werkzeug zum Einschrauben des auswechselbaren Glasfasereinsatzes in das Laserhandstück und als Behältnis für die Glasfaser während der Sterilisation.

Noch ein weiterer Gegenstand der Erfindung betrifft ein Steuergerät für ein Laserhandstück, umfassend einen mit dem Steuergerät verbunden Lichtleiter. Das Gehäuse weist einen umfangseitig umlaufenden Ringspalt auf, wobei der Ringspalt das Gehäuse in eine untere Gehäusehälfte und in eine obere Gehäusehälfte zumindest optisch teilt und wobei die obere Gehäusehälfte von vorne betrachtet seitlich über die untere Gehäusehälfte hervorsteht und die untere Gehäusehälfte von vorne betrachtet hinten seitlich über die obere Gehäusehälfte hervorsteht.

Damit ist die Zuleitung zum Handstück trotz einer Länge von ca. 2 Metern noch einfach handhabbar. Den unterschiedlichen räumlichen Bedingungen in der Zahnarztpraxis kann dadurch Rechnung getragen werden, dass der Anwender nicht die volle Länge der Zuleitung zum Handstück abwickeln muss.

Der Lichtleiter kann in einem mit dem Steuergerät verbundenen Handstückschlauch angeordnet sein. Dabei kann in dem Handstückschlauch auch zusätzlich eine Signalleitung oder eine Steuerleitung angeordnet sein, um eine Signalübertragung vom Laserhandstück an das Steuergerät zu ermöglichen.

Vorteilhafterweise kann die Breite des Ringspaltes mindestens dem einfachen Durchmesser und weniger als dem 2-fachen, vorzugsweise weniger als dem 1,2-fachen Durchmesser des Lichtleiters oder des Handstückschlauchs entsprechen, um ein definiertes Aufwickeln zu ermöglichen.

Vorzugsweise kann der Lichtleiter aus dem Gehäuseinneren knickfrei in den Grund des Ringspaltes geführt sein.

Gemäß einer Weiterbildung kann der Lichtleiter oder der Handstückschlauch elektrische und/oder optische Anschlüsse aufweisen und zu Wartungszwecke vom Steuergerät trennbar sein.

Weiterhin kann im Ringspalt eine Hemmung vorgesehen sein, die als mechanische Stopper, als Klettband oder als magnetischer Halter ausgebildet sein kann.

Am Gehäuse selbst kann eine Ablageeinrichtung für das Handstück vorgesehen sein, wobei insbesondere dann, wenn ein vorstehend beschriebenes Laserhandstück verwendet wird, eine Längenanpassung des Lichtleiters oder des Handstückschlauchs bei dem in der Ablage befindlichen Handstück möglich ist.

### Kurze Beschreibung der Zeichnung

In der Zeichnung ist Ausführungsbeispiel der Erfindung dargestellt. Es zeigt die:
- Fig. 1: Einen erfindungsgemäß Laserhandstück in einer perspektivischen Ansicht, die
- Fig. 2: einen im Laserhandstück aus Fig. 1 innenliegenden und verschiebbaren Grundkörper, die
- Fig. 3: einen Schnitt durch den Grundkörper aus Fig. 2, die
- Fig. 4: eine Explosionszeichnung einer Griffhülse des Laserhandstücks aus Fig. 1, die
- Fig. 5: einen Längsschnitt durch das Laserhandstück aus Fig. 1, die
- Fig. 6: eine Seitenansicht eines Steuergeräts für das Laserhandstück aus Fig. 1, die
- Fig. 7: einen schematischen Schnitt durch das Steuergerät aus Fig. 6, die
- Fig. 8: einen auswechselbaren Glasfasereinsatz mit einer Aufbewahrungshülse, die
- Fig. 9A: eine Ausgestaltung der Kabelaufwicklung des Steuergeräts aus Fig. 6, die
- Fig. 9B: eine Kabelhemmung mit einer mechanischen Sperre und die
- Fig. 9C: eine magnetische Kabelhemmung.

### Ausführungsbeispiele der Erfindung

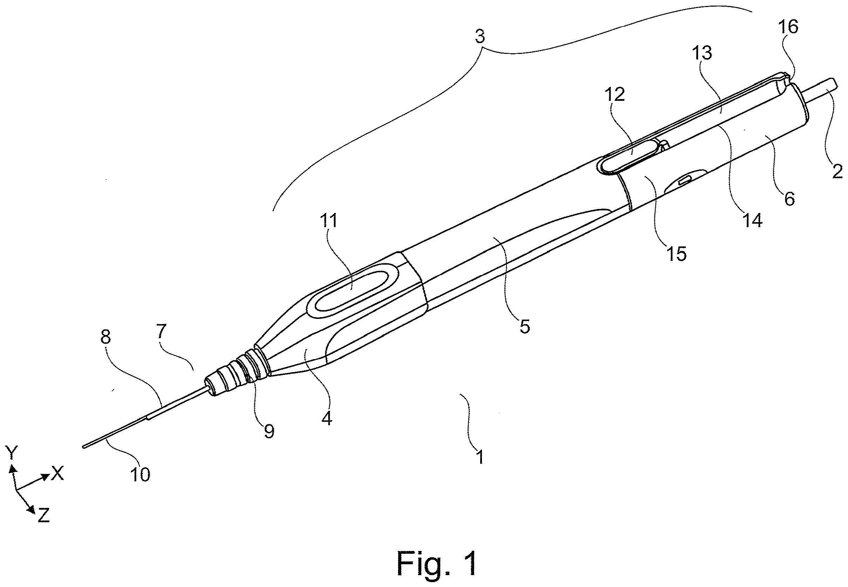

In Fig. 1 ist eine erfindungsgemäßes Laserhandstück für dentale Anwendungen in einer perspektivischen Darstellung gezeigt. Das Laserhandstück 1 weist einen Handstückschlauch 2 mit einem nicht dargestellten Lichtleiter und gegebenenfalls nicht dargestellten elektrischen Leitungen auf, wobei der Handstückschlauch 2 in einer Griffhülse 3 geführt ist. Die Griffhülse 3 kann mehrteilig ausgebildet sein und weist einen Fingerauflagebereich 4, einen Stützbereich 5 und einen Verstellbereich 6 auf, die in Längsrichtung gesehen hintereinanderliegend angeordnet sind. An dem dem Handstückschlauch 2 abgewandten Ende der Griffhülse 3 ist eine Spitze 7 mit einem Führungsröhrchen 8 und einem Anschlussteil 9 an die Griffhülse 3 vorgesehen. Aus dem Führungsröhrchen 8 der Spitze 7 tritt ein Applikationselement für Laserlicht in Form einer Glasfaser 10 aus, mittels derer das Laserlicht an den Ort der Behandlung geleitet wird. Derartige Glasfasern sind für die Behandlung von Weichgewebe und Hartgewebe bekannt. Nach jeder Benutzung des Laserhandstücks kann es erforderlich sein, die Glasfaser 10 zu kürzen, um in die Glasfaser 10 während der Bearbeitung eingebrannte Geweberückstände zu entfernen.

In Fig. 1 ist weiterhin zu erkennen, dass die Griffhülse 3 im Fingerauflagebereich 4 ein sich parallel zur Längsachse des Laserhandstücks erstreckendes Tastenfeld 11 aufweist, über welches auf einen darunter angeordnete, später erläuterte Tastschalter eingewirkt werden kann. Dieses Tastenfeld 11 ist zwischen 20 und 45 mm lang und ermöglicht die Betätigung des darunter liegenden Tastschalters unabhängig von dessen tatsächlicher Größe sowohl bei einer Frontzahnbehandlung, bei welcher das Laserhandstück weit vorne gegriffen wird, als auch bei einer Backenzahnbehandlung, bei das Laserhandstück weiter hinten gegriffen wird.

Weiterhin ist in Fig. 1 ein Verschiebeknopf 12 dargestellt, der in dem Verstellbereich 6 der Griffhülse 3 in einer Ausnehmung 13 angeordnet ist, wobei die Griffhülse eine Verschiebebahn 14 mit einer Führung für den Verschiebeknopf 12 aufweist. Der Verschiebeknopf 12 ist an einem in Inneren der Griffhülse 3 angeordneten hier nicht dargestellten Grundkörper befestigt, wobei dieser Grundkörper mittels des Verschiebeknopfs 12 entlang der Verschiebebahn 14 in der Griffhülse 3 längs verschiebbar ist. Das Zusammenwirken des Verschiebeknopfs 12 und der Führungsbahn 14 verhindert ein Verdrehen gegenüber der Griffhülse 3.

Der Verstellbereich 6 der Griffhülse 3 ist als separates Bauteil in Form einer Endhülse 15 ausgebildet und weist im Bereich der Ausnehmung 13 eine mit dieser verbundene endseitige Ausnehmung 16 auf, in welche der Handstückschlauch 2 einlegbar ist.

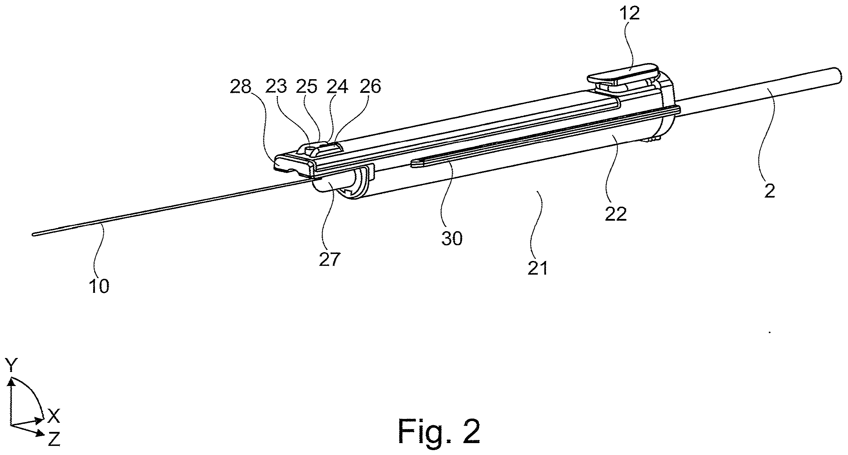

In Fig. 2 ist der vorstehend erwähnte, im Inneren der Griffhülse 3 aus Fig. 1 angeordnete Grundkörper 21 dargestellt, an dessen dem Handstückschlauch 2 zugewandten Ende der Verschiebeknopf 12 vorgesehen ist und an dessen gegenüberliegendem Ende die Glasfaser 10 angeordnet ist. Der Verschiebeknopf 12 ist auf der Oberseite des Gehäuses 22 des Grundkörpers 21 angeordnet, ebenso sind zwei Tastschalter 23, 24 vorgesehen, die in Längsrichtung gesehen versetzt zueinander angeordnet sind und die durch seitlich angeordnete Stege 25, 26 gegen ein unbeabsichtigtes Betätigen der Tastschalter 23, 24 gesichert sind. Die Höhe und der Abstand der Stege 25, 26 sind so bemessen, dass der in dem anzuwendenden Standard IEC 60601-1 beschriebene Normfinger eine Betätigung nicht erlaubt.

Die am Grundkörper 21 angeordnete Glasfaser 10 weist ein auch als Ferrule bezeichnetes Anschlussstück 27 auf, welches in dem Gehäuse 22 beispielsweise über ein Schraubgewinde lösbar befestigt ist, um im Falle des Verschleißes oder des Bruchs der Glasfaser 10 ein Auswechseln zu ermöglichen. Die Glasfaser 10 und das Anschlussstück 27 bilden zusammen den auswechselbaren Glasfasereinsatz. Die Oberseite des Gehäuses 22 überragt dieses Anschlussstück 27 nach vorne, so dass an der dem Anschlussstück 27 zugewandten Seite des Gehäusevorsprungs 28 ein Sensor vorgesehen sein kann, der das Vorhandensein des auswechselbaren Glasfasereinsatzes feststellt. Dies geschieht beispielsweise durch eine Erkennung von Licht im Bereich der Wellenlänge eines Führungslaserstrahls oder des Bearbeitungslaserstrahls, in dem beispielsweise die Intensität dieser Wellenlängen als Signal erfasst wird.

Die Führung des Grundkörpers in der Griffhülse kann auch über zusätzliche Nuten und Nocken erfolgen, wobei hier am Grundkörper ein längs verlaufender Nocken 30 erkennbar ist.

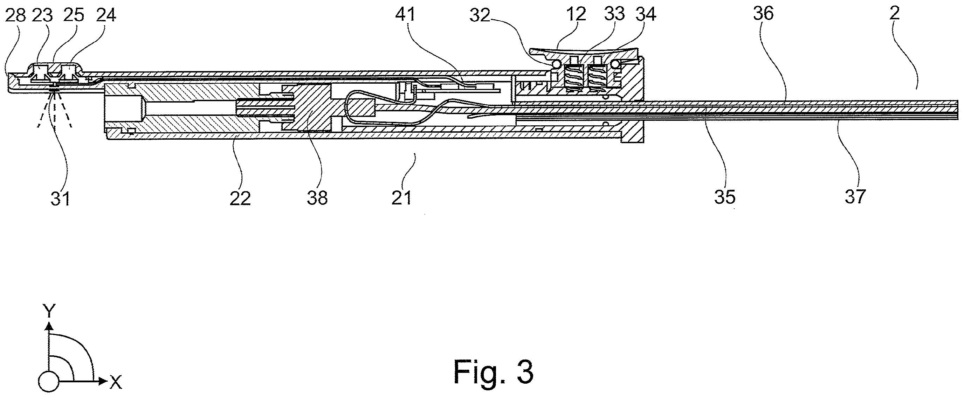

In Fig. 3 ist ein Schnitt längs durch den Grundkörper aus Fig. 2 dargestellt, wobei jedoch der auswechselbare Glasfasereinsatz weggelassen ist. Aus diesem Grund wird ein an der Unterseite des Vorsprungs 28 angeordneter Sensor 31 dann ein Fehlersignal liefern, wenn aus dem Gehäuse 22 des Grundkörpers 21 Laserstrahlen austreten.

Der Grundkörpers 21 ist mit dem an der Oberseite des Gehäuses 22 angeordneten Verschiebeknopf 12 versehen, der gegenüber dem Gehäuse 22 federnd eindrückbar gelagert ist und der einen umlaufenden O-Ring 32 aufweist, welcher mit der Verschiebebahn der Griffhülse aus Fig. 1 zum Zweck der Hemmung der Verschiebung zusammenwirkt. Durch Eindrücken des Verschiebeknopfs 12 wird der O-Ring 32 gegenüber der Verschiebebahn gelöst, sodass ein Verschieben begünstigt ist. Beim Loslassen des Verschiebeknopfs 12 wird dieser durch Federelemente 33, 34 gegen das Gehäuse 22 an die Verschiebebahn gedrückt.

Im Bereich des Verschiebeknopfs 12 mündet auch der Handstückschlauch 2 in das Gehäuse 22 ein und ist dort mittels einer Zugentlastung gehalten. Der Handstückschlauch 2 weist dabei zum einem einen Lichtleiter 35 auf, zum anderen Leitungen 36, wobei der Lichtleiter 35 und die Leitungen 36 von einer gemeinsamen Hülle 37 umschlossen sind. Bei den Leitungen 36 kann es sich um Signal- oder Steuerleitungen handeln, es können aber auch weitere Lichtleiter zur Signalübertragung vorhanden sein.

Im Inneren des Gehäuses 22 wird der Lichtleiter 35 bis zu einem optischen Lichteinkoppler 38 geführt und tritt aus dem optischen Lichteinkoppler 38, der auch optische Bauteile enthalten kann, wieder zum Applikationselement hinaus.

Das Gehäuse 22 weist darüber hinaus eine Leiterplatte 41 auf, an der die elektrischen Leitungen 36 kontaktiert sind und die mit den Tastschaltern 23, 24 und dem Sensor 31 elektrisch leitend verbunden ist. Dabei kann auch eine Auswerteeinheit auf der Leiterplatte 41 enthalten sein, um die von den Tastschaltern 23, 24 und dem Sensor 31 stammenden elektrischen Signale auszuwerten und die Bereitstellung des Laserlichts durch die Steuereinheit zu erlauben oder zu verhindern. Die Leiterplatte 41 kann auch lediglich zur Weiterleitung von Signalen an das Steuergerät dienen, wenn die Auswerteeinheit dort untergebracht ist. Die Signale können elektrisch oder optisch übertragen werden.

Das Gehäuse 22 des Grundkörpers ist hermetisch gegen die Umgebung abgedichtet.

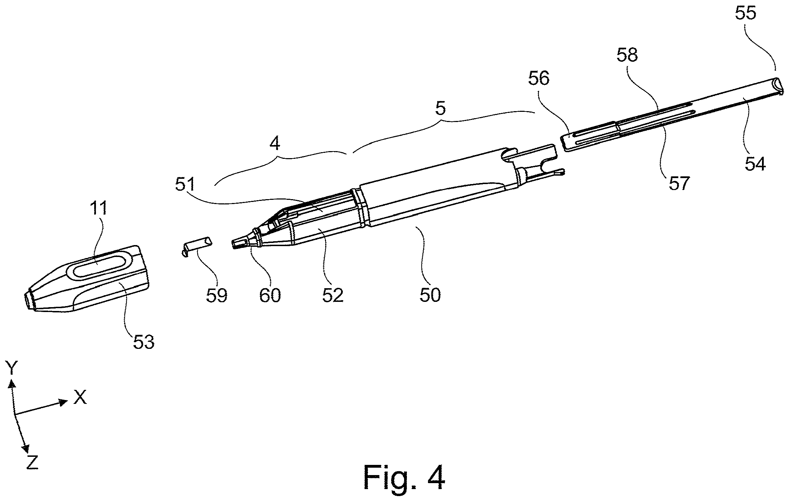

In Fig. 4 ist die Griffhülse 3 im Fingerauflagebereich 4 und Stützbereich 5 als Explosionszeichnung dargestellt, wobei im Fingerauflagebereich 4 an dem Hülsenkörper 50 eine Aussparung 51 vorgesehen ist, über welche die Tastschalter am nicht dargestellten Grundkörper aus Fig. 2 betätigbar sind. Dazu weist das vordere Ende 52 des Hülsenkörpers 50 einerseits einen abnehmbaren Überzug 53 mit einem verformbaren Tastenfeld 11 auf, andererseits ist ein Hebel 54 vorgesehen, der im Inneren des Hülsenkörpers 50 verläuft und die Ausnehmung 51 überdeckt. Der Hebel 54 ist nach dem Zusammenbau mit einem Drehgelenk 55 im Hülsenkörper 50 gelagert und das vordere Ende 56 liegt vollständig unter dem Tastenfeld 11. Damit der Hebel auf die Tastschalter des Grundkörpers aus Fig. 2 heruntergedrückt werden kann, sind Längsnuten 57, 58 vorgesehen, deren Breite so bemessen ist, dass die Sicherungsstege 25, 26 aus Fig. 2 dort eingreifen.

Um den Hebel 54 sicher in die abgehobene Endstellung zu bringen, ist weiterhin ein Federelement 59 vorgesehen, das im vorderen Teil 52 gehalten und mit dem Hebel 55 verbunden ist. Dieses Federelement 59 ist nicht zwingend erforderlich, sorgt aber für eine zusätzliche Sicherheit bei der Rückstellung des Hebels 54 über die Rückstellung durch die Tastschalter selbst hinaus.

Das Ende des Vorderteils 52 ist als Luer-Befestigung ausgebildet, damit ein Standardanschluss 60 zur Befestigung der nicht dargestellten auswechselbaren Spitze 7 aus Fig. 1 verwendet werden kann. Derartige Befestigungen sind im medizinischen Bereich Stand der Technik.

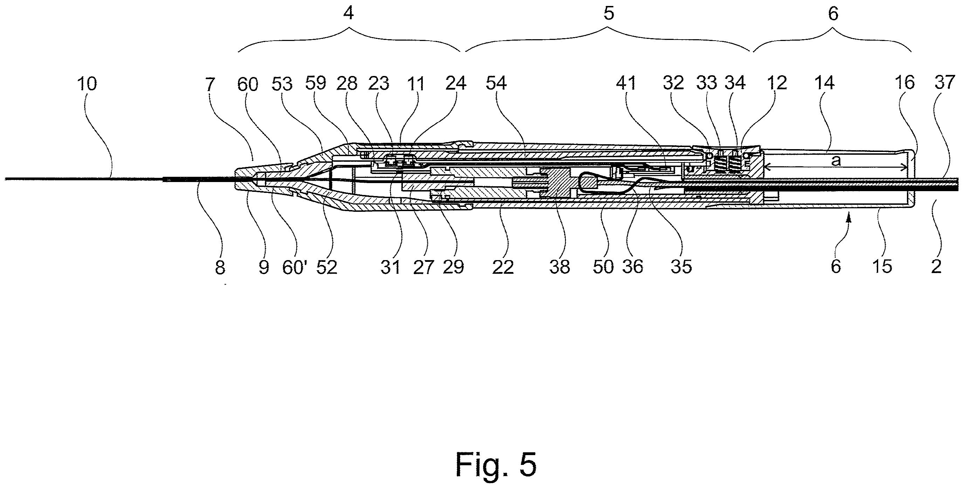

In Fig. 5 ist das zusammengebaute Laserhandstück aus den Fig. 1-4 im Längsschnitt dargestellt. Das Führungsröhrchen 8 der Spitze 7 ist fest im Anschlussteil 9 gehalten und der Anschlussteil 9 ist mit einem Luer-Anschluss 60' an das vordere Ende 52 des Hülsenkörpers 50 aus Fig. 4 ausgebildet, das ebenfalls einen entsprechenden Luer-Standardanschluss 60 aufweist.

Das Führungsröhrchen 8 kann bei eingebrachter Glasfaser mittels eines Biegewerkzeugs gebogen werden, ohne die eingebrachte Glasfaser zu beschädigen. Diese Biegung kann jedoch nicht rückgängig gemacht werden, außerdem ist es nicht möglich, die Glasfaser 10 durch die Spitze 7 zu schieben, wenn das Führungsröhrchen 8 abgebogen ist.

Die weiteren angegebenen Bauteile entsprechen denen aus den Fig. 1 - 4, wobei darauf hingewiesen wird, dass die Glasfaser 10 in eine maximal vorgeschobene Endstellung des Gehäuses 22 des Grundkörpers bezüglich des Hülsenkörpers 50 gebracht ist und damit um die maximale Länge über das Führungsröhrchen heraussteht. Beim Zurückschieben des Gehäuses 22 des Grundkörpers in der Endhülse 15 mittels des Verschiebeknopfs 12, der dazu eingedrückt wird, kann das Gehäuse 22 einen Verstellweg a zurücklegen. Aufgrund der Ausnehmung 16 am schlauchseitigen Ende des Endstücks 15 wird der Handstückschlauch 2 um diese Länge a aus dem Laserhandstück geschoben.

In dieser Schnittdarstellung ist auch der auf der Unterseite des Vorsprungs 28 angeordnete und auf den auswechselbaren Glasfasereinsatz, der aus der Glasfaser 10 und dem Anschlussstück 27 besteht, ausgerichtete Sensor 31 zur Detektion von Laserstrahlung erkennbar, der mit der Leiterplatte 41 elektrisch verbunden ist.

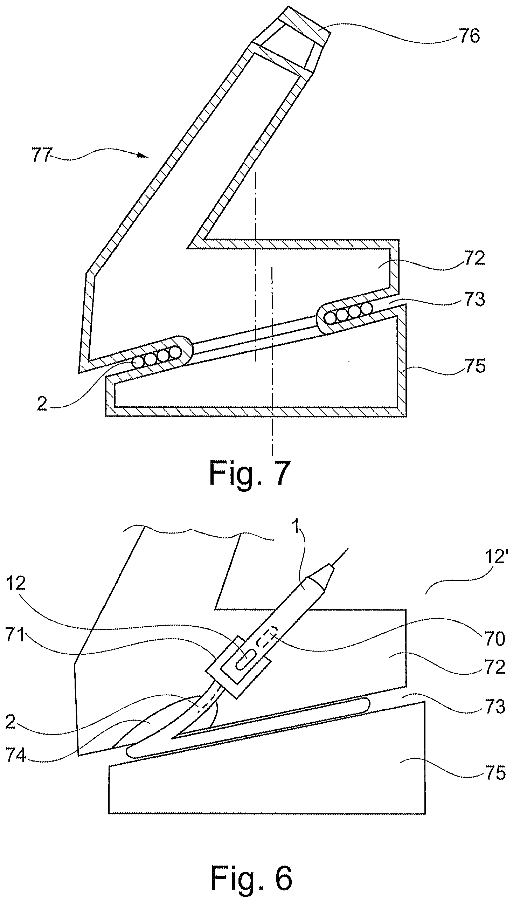

In Fig. 6 ist ein Steuergerät zur Versorgung des vorstehend beschriebenen Laserhandstücks dargestellt, wobei das Laserhandstück 1 in einer Ablage 71 an einem Gehäuseteil 72 gehalten ist. Der Handstückschlauch 2 ist dabei in einen Ringspalt 73 eingelegt und verlässt diesen Ringspalt 73 erst kurz vor der Ablage 71. Um ein Abknicken zu vermeiden, kann das Gehäuseteil 72 eine Mulde 74 aufweisen. Mittels des Verschiebeknopfs 12 am Laserhandstück 1 kann die Schlauchlänge 2, die aus dem Ringspalt 73 herausgeführt ist, durch Verschieben in eine Position 12' gespannt werden, sodass der Handstückschlauch 2 auch beim Transport des Steuergeräts 70 nicht aus dem Ringspalt 73 fällt.

Bereits in Fig. 6 ist ersichtlich, dass der obere Gehäuseteil 72 des Steuergeräts 70 gegenüber dem unteren Gehäuseteil 75 versetzt angeordnet ist, wobei in der hier gewählten Seitenansicht auf die rechte Seite der Steuereinheit der obere Gehäuseteil 72 vorne, also in der Zeichnung auf der linken, dem Benutzer zugewandten Seite über das untere Gehäuseteil 75 hervorsteht, wohingegen auf der dem Benutzer abgewandten Seite, dass ist in der Zeichnung rechts, das untere Gehäuseteil 75 über das obere Gehäuseteil 72 hervorsteht. Dies wird beispielsweise dann erreicht, wenn ein Zylinder quer geschnitten wird und beide Zylinderteile zueinander einen Versatz aufweisen. Diese besondere Ausführung des Gehäuses des Steuergeräts hat den Vorteil, dass beim Aufwickeln des Handstückschlauchs 2 ein Auflegen des Handstückschlauchs 2 auf das untere Gehäuseteil 75 im vom Benutzer abgewandten Bereich des Steuergeräts möglich ist, wohingegen der Handstückschlauch an der dem Benutzer zugewandten Seite des Steuergeräts unter dem oberen Gehäuseteil hindurch geführt wird und beim Herumführen jeweils in die umlaufenden Ringspalt 73 eingelegt und damit aufgewickelt wird.

In dem in Fig. 7 schematisch dargestellten Schnitt wird deutlich, dass der Handstückschlauch 2 in dem Ringspalt 73 spiralförmig aufgewickelt ist, sodass jede Windung an der vorhergehenden anliegt. Bei festsitzender Aufwicklung ist der Handstückschlauch 2 daher immer in derselben Position in dem Ringspalt 73 abgelegt und das aus dem Ringspalt 73 herausragende Ende des in der Ablage 71 aus Fig. 5 abgelegten Handstücks ist immer gleich lang.

An dem Gehäuseteil 72 kann eine Anzeigevorrichtung angebracht sein, die hier in Form eines Pultes 77 ausgeführt ist, ebenso können nicht dargestellte Eingabemittel wie Tasten oder Drehregler vorgesehen sein, um eine nicht dargestellte, im Inneren des Gehäuses angeordnete Vorrichtung zur Bereitstellung von Laserstrahlen zu steuern. Weiterhin kann ein oberhalb des Pults 77 ein Griff 76 vorgesehen sein, um das Steuergerät einfach zu transportieren.

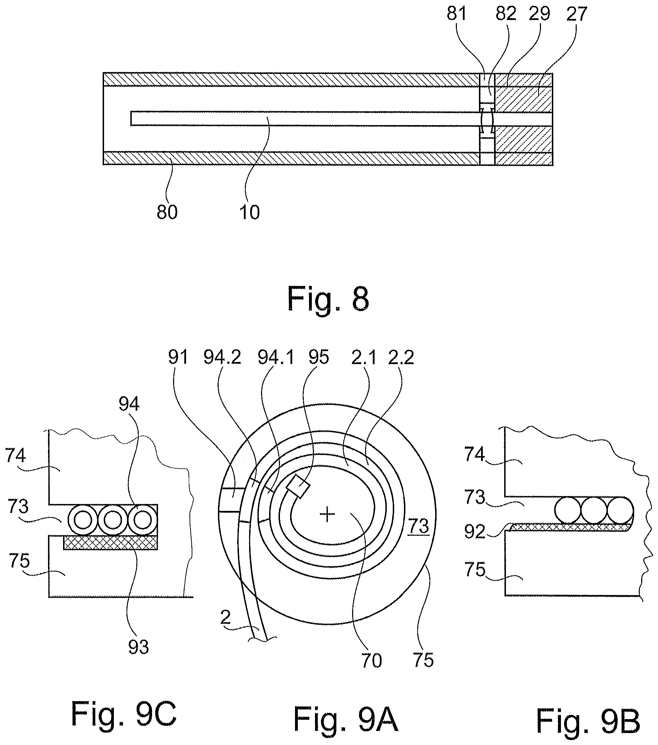

In Fig. 8 ist ein auswechselbarer Glasfasereinsatz dargestellt, wobei die Glasfaser 10 in dem Anschlussstück 27 befestigt ist und das Anschlussstück 27 einen mit einem Außengewinde 29 versehenen Halteabschnitt aufweist, zusammen auch bezeichnet als eine Wechselfieber. Anstelle eines Außengewindes 29 kann am Halteabschnitt auch ein Bajonettverschluss vorgesehen sein oder ein sonstiger Befestigungsmechanismus der durch Verdrehen des Anschlussstücks 27 eine Verbindung bewirkt.

Um das Anschlussstück 27 ohne Beschädigung der Glasfaser 10 befestigen zu können, etwa durch Einschrauben oder durch Verdrehen, ist eine Aufsteckhülse 80 vorgesehen, die sich um die Glasfaser 10 herum erstreckt und die einen Verbindungsbereich 81 zu einem Drehmomentübertragungsbereich 82 am Anschlussstück 27. Bei dem Drehmomentübertragungsbereich 82 kann es sich etwa um eine Art Sechskant-Mutter für einen Steckschlüssel handeln, noch besser handelt es sich aber um eine Verbindung mit lediglich punktförmigen Auflageflächen, etwa einer einseitigen Abflachung oder um einem z. B. senkrecht zur Längsachse eingebrachten Stift. Auch ein zum Festschrauben auf Reibschluss ausgelegter rotationssymmetrischer Drehmomentübertragungsbereich ist anwendbar. Es ist die Lösung zu bevorzugen, die beim Festschrauben die Möglichkeit bietet, das Drehmoment beim Festschrauben einfach zu begrenzen.

Die Aufsteckhülse 80 mit gleichzeitiger Werkzeugausformung dient einerseits als Transportschutz für die Glasfaser, als Werkzeug zum Einschrauben des auswechselbaren Glasfasereinsatzes in den Grundkörper 21 aus Fig. 2 und als Behältnis für die Glasfaser während der Sterilisation.

In Fig. 9A - 9C sind verschiedene Möglichkeiten einer Hemmung gezeigt, die eine windungsweise Abwicklung des Handstückschlauchs 2 ermöglichen. In Fig. 9A sind zunächst zwei Windungen 2.1, 2.2 des im Gehäuse vorgesehenen Ringspalts 73 des Steuergeräts 70 aufgewickelten Handstückschlauchs 2 dargestellt, wobei ein Halteelement 91 in einer Stellung bei neun Uhr vorgesehen ist. Durch dieses Halteelement 91 wird verhindert, dass sich die aufgewickelten Windungen 2.1, 2.2 des Handstückschlauchs 2 unbeabsichtigt lockern. Die kann dadurch erreicht werden, dass im Ringspalt 73 zwischen dem oberen und dem unteren Gehäuseteil 74, 75 als Halteelement 91 ein Silikonwulst oder eine elastische Auflage 92 vorgesehen ist. Alternativ dazu kann, wie in Fig. 9C dargestellt, ein unterhalb des Ringspalts 73 im unteren Gehäuseteil 75 angeordneter Magnet 93 als Halteelement vorgesehen sein, der mit einer magnetischen Manschette 94 am Handstückschlauch 2, dargestellt in Fig. 9A als Schlauchmanschette 94.1 und 94.2, zusammenwirkt.

Anstelle des Magnets und der Hülsen kann auch eine Klettverbindung vorgesehen sein.

Der Lichtleiter oder der Handstückschlauch wird vom Innern des Steuergeräts 70 knickfrei in den Ringraum 73 geführt und ist im Innern über einen Anschluss 95 mit dem Steuergerät 70 verbunden. Über den Anschluss 95 kann der Lichtleiter 35 oder der Handstückschlauch 2 zu Reparatur- oder Wartungszwecken vom Steuergerät getrennt werden.

### Bezugszeichenliste

- 1: Laserhandstück
- 2: Handstückschlauch
- 2.1: Windung
- 2.2: Windung
- 3: Griffhülse
- 4: Fingerauflagebereich
- 5: Stützbereich
- 6: Verstellbereich
- 7: Spitze
- 8: Führungsröhrchen
- 9: Anschlussteil
- 10: Glasfaser - Applikationselement
- 11: Tastenfeld
- 12, 12': Verschiebeknopf
- 13: Ausnehmung
- 14: Verschiebebahn
- 15: Endhülse
- 16: Ausnehmung
- 21: Grundkörper
- 22: Gehäuse
- 23: Tastschalter
- 24: Tastschalter
- 25: Steg/Sicherungssteg
- 26: Steg/Sicherungssteg
- 27: Anschlussstück
- 28: Vorsprung
- 29: Außengewinde / Halteabschnitt
- 30: Nocken
- 31: Sensor
- 32: O-Ring
- 33: Federelement
- 34: Federelement
- 35: Lichtleiter
- 36: Leitung / Signalleitung / Steuerleitung
- 37: Hülle
- 38: Lichteinkoppler
- 41: Leiterplatte / Auswerteeinheit
- 50: Hülsenkörper
- 51: Aussparung/Ausnehmung
- 52: vorderes Ende/vorderer Teil
- 53: Überzug
- 54: Hebel
- 55: Drehgelenk/Hebel
- 56: vorderes Ende
- 57: Längsnut
- 58: Längsnut
- 59: Federelement
- 60: Standardanschluss Luer am Gehäuse
- 60': Luer-Anschluss an der Spitze
- 70: Steuergerät
- 71: Ablage
- 72: Gehäuseteil
- 73: Ringspalt
- 74: Mulde
- 75: Unterteil/Gehäuseteil
- 76: Griff
- 77: Pult
- 80: Aufsteckhülse
- 81: Verbindungsbereich
- 82: Drehmomentübertragungsbereich
- 83: Halteabschnitt
- 91: Halteelement
- 92: Auflage
- 93: Magnet
- 94: Manschette
- 94.1: Schlauchmanschette
- 94.2: Schlauchmanschette
- 95: Anschluss für Lichtleiter / Handstückschlauch
- a: Verschiebeweg

## Patentansprüche

1. Laserhandstück, umfassend einen Lichtleiter (35), **dadurch gekennzeichnet, dass** der Lichtleiter (35) an einer Lichtkopplungsstelle (38) in einem Grundkörper (21) befestigt ist, dass ein Glasfasereinsatz (10,27) für Laserlicht mit der Lichtkopplungsstelle (38) auswechselbar verbunden ist und dass der Grundkörper (21) in einer Griffhülse (3) axial verstellbar gehalten ist, wobei der Grundkörper (21) vollständig innerhalb der Griffhülse (3) angeordnet ist.

2. Laserhandstück nach Anspruch 1, **dadurch gekennzeichnet, dass** die Griffhülse (3) mehrteilig ist und einen Fingerauflagebereich (4), einen Stützbereich (5) und einen Verstellbereich (6) aufweist.

3. Laserhandstück nach Anspruch 1 oder 2, **dadurch gekennzeichnet, dass** ein Tastschalter (23, 24) zum Anschalten des Laserlichts vorgesehen ist, der vorzugsweise über ein Tastenfeld (11) mit einer Länge von mindestens 20 mm bedienbar ist.

4. Laserhandstück nach Anspruch 3, **dadurch gekennzeichnet, dass** der Tastschalter (23, 24) am Grundkörper (21) angebracht ist und von einem an der Griffhülse (3) angebrachten schwenkbaren Hebel (54) abgedeckt ist.

5. Laserhandstück nach Anspruch 3 oder 4, **dadurch gekennzeichnet, dass** der Tastschalter (23, 24) am Grundkörper (21) gegen unbeabsichtigtes Betätigen bei abgezogener Griffhülse (3) gesichert ist, in dem die Sicherung des Tastschalters (23, 24) durch eine Erhebung, insbesondere durch in Längsrichtung verlaufende seitliche Stege (25, 26) gebildet ist, deren Höhe und Abstand zueinander so gewählt ist, so dass versehentliches Auslösen durch den Anwender nicht möglich ist.

6. Laserhandstück nach einem der Ansprüche 3 bis 5, **dadurch gekennzeichnet, dass** mehrere redundante Tastschalter (23, 24) vorgesehen sind.

7. Laserhandstück nach einem der Ansprüche 3 bis 6, **dadurch gekennzeichnet, dass** der Grundkörper (21) einen auf den Lichtweg im Grundkörper (21) ausgerichteten Sensor (31) aufweist, der Licht mit der Wellenlänge des über den Lichtleiter (35) zugeführten Lichts erkennt und dass ein Signal an eine Auswerteeinheit (41) übermittelbar ist.

8. Laserhandstück 1 nach einem der Ansprüche 1 bis 7, **dadurch gekennzeichnet, dass** der Glasfasereinsatz (10,27) ein an einem Ende angeordnetes Anschlussstück (27) zum Anschluss an das Laserhandstück (1) und eine sich von dem Anschlussstück (27) weg erstreckende Glasfaser (10) mit einem dem Anschlussstück (27) abgewandten Bearbeitungsende aufweist, wobei das Anschlussstück (27) einen Abschnitt (82) zur Einleitung und Übertragung eines Drehmoments auf einen Halteabschnitt (29) aufweist, wobei eine die Glasfaser (10) umgebende Hülse vorgesehen ist, die auf den Abschnitt (82) zur Einleitung und Übertragung eines Drehmoments des Anschlussstücks (27) aufsteckbar ist und deren Ende einen Verbindungsbereich (81) aufweist, der mit dem den Abschnitt (82) zur Einleitung und Übertragung eines Drehmoments des Anschlussstücks (27) zusammenwirkt.

## Claims

1. A laser handpiece, comprising a light guide (35), **characterised in that** the light guide (35) is attached to a light coupling point (38) in a base body (21), and **in that** a glass fibre insert (10, 27) for laser light is exchangeably connected to the light coupling point (38) and **in that** the base body (21) is held so as to be axially adjustable in a grip sleeve (3), wherein the base body (21) is arranged completely within the grip sleeve (3).

2. The laser handpiece according to claim 1, **characterised in that** the grip sleeve (3) is in several parts and has a finger support area (4), a support area (5) and an adjustment area (6).

3. The laser handpiece according to claim 1 or 2, **characterised in that** a pushbutton switch (23, 24) is provided for switching on the laser light, which can preferably be operated via a keypad (11) with a length of at least 20 mm.

4. The laser handpiece according to claim 3, **characterised in that** the push button switch (23, 24) is attached to the base body (21) and is covered by a pivotable lever (54) attached to the grip sleeve (3).

5. The laser handpiece according to claim 3 or 4, **characterised in that** the pushbutton switch (23, 24) on the base body (21) is secured against unintentional activation when the grip sleeve (3) is removed, wherein the locking device of the pushbutton switch (23, 24) is formed by an elevation, in particular by lateral stop ridges (25, 26) running in a longitudinal direction, the height and spacing of which are selected so that accidental triggering by the user is not possible.

6. The laser handpiece according to one of claims 3 to 5, **characterised in that** a plurality of redundant pushbutton switches (23, 24) are provided.

7. The laser handpiece according to one of claims 3 to 6, **characterised in that** the base body (21) has a sensor (31) that is aligned with the light path in the base body (21) and that detects and recognises light with the wavelength of the light supplied via the light guide (35), and **in that** a signal can be transmitted to an evaluation unit (41).

8. The laser handpiece 1 according to one of claims 1 to 7, **characterised in that** the glass fibre insert (10, 27) has a connecting piece (27) arranged at one end for connection to the laser handpiece (1) and a glass fibre (10) extending away from the connecting piece (27) with a machining end facing away from the connection piece (27), wherein the connection piece (27) has a section (82) for introducing and transmitting a torque to a holding section (29), wherein a sleeve surrounding the glass fibre (10) is provided that can be plugged onto the section (82) for the introduction and transmission of a torque of the connection piece (27) and the end of which has a connecting region (81) that interacts with the section (82) for the introduction and transmission of a torque of the connection piece (27).

## Revendications

1. Pièce à main laser comprenant un guide de lumière (35), **caractérisée en ce que** le guide de lumière (35) est fixé à un point de couplage de lumière (38) dans un corps de base (21) **en ce qu'**un insert de fibre de verre (10, 27) pour la lumière laser est relié de manière interchangeable au point de couplage de lumière (38) et **en ce que** le corps de base (21) est maintenu réglable axialement dans un manchon de préhension (3), le corps de base (21) étant disposé entièrement à l'intérieur du manchon de préhension (3).

2. Pièce à main laser selon la revendication 1, **caractérisée en ce que** le manchon de préhension (3) est en plusieurs parties et présente une zone de positionnement des doigts (4), une zone d'appui (5) et une zone de réglage (6).

3. Pièce à main laser selon la revendication 1 ou 2, **caractérisée en ce qu'**un bouton-poussoir (23, 24) sert à allumer la lumière laser, qui peut être actionné de préférence par un clavier (11) d'une longueur d'au moins 20 mm.

4. Pièce à main laser selon la revendication 3, **caractérisée en ce que** le bouton-poussoir (23, 24) est fixé au corps de base (21) et est recouvert d'un levier pivotant (54) fixé au manchon de préhension (3).

5. Pièce à main laser selon la revendication 3 ou 4, **caractérisée en ce que** le bouton-poussoir (23, 24) sur le corps de base (21) est protégé contre un actionnement involontaire lors du retrait du manchon de préhension (3) par sécurisation du bouton-poussoir (23, 24) au moyen d'une saillie, en particulier des éléments de liaison latéraux (25, 26) s'étendant dans le sens longitudinal, dont la hauteur et l'écartement sont choisis de manière à rendre impossible un déclenchement accidentel par l'utilisateur.

6. Pièce à main laser selon l'une des revendications 3 à 5, **caractérisée en ce que** plusieurs boutons-poussoirs redondants (23, 24) sont prévus.

7. Pièce à main laser selon l'une des revendications 3 à 6, **caractérisée en ce que** le corps de base (21) présente un capteur (31) qui est aligné avec le trajet lumineux dans le corps de base (21) et qui reconnaît la lumière ayant la longueur d'onde de la lumière fournie via le guide de lumière (35) et **en ce qu'**un signal peut être transmis à une unité d'évaluation (41).

8. Pièce à main laser 1 selon l'une des revendications 1 à 7, **caractérisée en ce que** l'insert de fibre de verre (10, 27) présente une pièce de raccordement (27) disposée à une extrémité pour se relier à la pièce à main laser (1) et une fibre de verre (10) s'éloignant de la pièce de raccordement (27) dont une extrémité d'usinage se trouve à l'opposé de la pièce de raccordement (27), la pièce de raccordement (27) ayant une section (82) servant à introduire et transmettre un couple à une section de maintien (29), un manchon entourant la fibre de verre (10) étant prévu, lequel pouvant s'enficher sur la section (82) pour introduire et transmettre un couple de la pièce de raccordement (27) et dont l'extrémité présente une zone de liaison (81) qui coopère avec la section (82) pour introduire et transmettre un couple de la pièce de raccordement (27).
